(19) Europäisches Patentamt
European Patent Office
Office européen des brevets

(11) Veröffentlichungsnummer : 0 297 570 B1

# (12) EUROPÄISCHE PATENTSCHRIFT

(45) Veröffentlichungstag der Patentschrift : **05.06.91 Patentblatt 91/23**

(51) Int. Cl.$^5$ : **C07C 211/52,** C07C 233/15

(21) Anmeldenummer : **88110440.0**

(22) Anmeldetag : **30.06.88**

## (54) Derivate des 2,2-Bis-(3-aminophenyl)-hexafluorpropans und Verfahren zur Herstellung des 2,2-Bis-(3,4-diaminophenyl)-hexafluorpropans.

Die Akte enthält technische Angaben, die nach dem Eingang der Anmeldung eingereicht wurden und die nicht in dieser Patentschrift enthalten sind.

(30) Priorität : **02.07.87 DE 3721839**

(43) Veröffentlichungstag der Anmeldung : **04.01.89 Patentblatt 89/01**

(45) Bekanntmachung des Hinweises auf die Patenterteilung : **05.06.91 Patentblatt 91/23**

(84) Benannte Vertragsstaaten : **BE CH DE FR GB IT LI NL**

(56) Entgegenhaltungen : **US-A- 3 310 573**

(56) Entgegenhaltungen : **CHEMICAL ABSTRACTS, Band 87, Nr. 23, 5. Dezember 1977, Seite 577, Zusammenfassung Nr. 184125v, Columbus, Ohio, US; B.R: LIVSHITS et al.: "Synthesis of hexafluoroisopropylidene-m,m'-diphenyl diisocyanate"**
**The soviet chemical industry, 9:7, (1977), s. 584/585.**
**Lehrbuch der organische Chemie, 20. Auflage, 1984.**

(73) Patentinhaber : **HOECHST AKTIENGESELLSCHAFT**
**Postfach 80 03 20**
**W-6230 Frankfurt am Main 80 (DE)**

(72) Erfinder : **Lau, Jürgen Dr.**
**Thüringer Weg 2**
**W-6238 Hofheim am Taunus (DE)**
Erfinder : **Siegemund, Günter Dr.**
**Frankfurter Strasse 21**
**W-6238 Hofheim am Taunus (DE)**

EP 0 297 570 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft neue Derivate des 2,2-Bis-(3-aminophenyl)-hexafluorpropans und ein neues Verfahren zur Herstellung von 2,2-Bis-(3,4-diaminophenyl)-hexafluorpropan (1) (Formel siehe Patentanspruch 4), einem fluorhaltigen Monomeren für Polybenzimidazole.

Es ist aus der US-PS 3 310 573 bekannt, Toluol mit Hexafluoraceton in Gegenwart von Flußsäure zu 2,2-Bis-(4-methylphenyl)-hexafluorpropan (2) umzusetzen. Dieses wird mit Chrom-(III)-oxid zur Dicarbonsäure (3) oxidiert, welche nachfolgend durch eine Schmidt-Reaktion mit Natriumazid/Schwefelsäure in das 2,2-Bis-(4-aminophenyl)-hexafluorpropan (4) umgewandelt wird. In den nächsten Reaktionsschritten wird (4) mit Acetanhydrid acetyliert und dann mit 70%iger Salpetersäure in konzentrierter Schwefelsäure in der 3-Stellung nitriert. Anschließend erfolgt die Abspaltung der Acetylgruppe, wozu die nitrierte Substanz in konzentrierter Schwefelsäure gelöst und mit Wasser versetzt wird, bis sich das 2,2-Bis-(3-nitro-4-aminophenyl)-hexafluorpropan (5) aus der Lösung ausscheidet.

Die weitere Umsetzung, d.i. die Reduktion, des 2,2-Bis-(3-nitro-4-aminophenyl)-hexafluorpropans (5) zu dem Tetramin (1) und die physikalischen Eigenschaften von (1) werden weder in der genannten US-PS 3 310 573 noch sonst beschrieben. Weiterhin lassen die in der US-PS zu den einzelnen Stufen gemachten Angaben nur für die Nitrierung eine Berechnung der Ausbeute zu.

Aus Chemical Abstracts, 1977, 87, Nr. 23, Seite 577, Nr. 184125v war weiterhin ein Verfahren zur Herstellung von 2,2-Bis-(3-amino-phenyl)-hexafluorpropan bekannt, wonach aus 2,2-Bis-(4-amino-phenyl)-hexafluorpropan durch Acetylierung, Nitrierung, Abspaltung der Acetylschutzgruppe, anschließende Diazotierung der Aminogruppe sowie Verkochung und nachfolgende Reduktion der Nitrogruppen diese Verbindung erhalten wird. Ein Weg zur Herstellung des erfindungsgemäß angestrebten Tetramins (1) wird dadurch nicht aufgezeigt.

2,2-Bis-(3-nitro-4-aminophenyl)-hexafluorpropan entsteht nach diesem Verfahren als Zwischenprodukt in insgesamt etwa 70%iger Ausbeute. Bei Einsatz des günstig zugänglichen 2,2-Bis-(3-aminophenyl)-hexafluorpropans als Ausgangsverbindung war aufgrund der Orientierungsregeln bei der elektrophilen Substitution am Benzolkern mit einer wesentlich geringeren Ausbeute an dem entsprechenden 2,2-Bis-(3-amino-4-nitrophenyl)-hexafluorpropan und dem Entstehen von technisch kaum abtrennbaren Nebenprodukten zu rechnen.

Für die etwaige Verwendung des Tetramins (1) als Monomerbaustein für Polymere ist es nun wichtig, daß diese Verbindung, die in Lösung extrem oxidationsempfindlich ist, leicht und in reiner Form zugänglich ist.

Das in der 3. Stufe des vorgenannten Verfahrens hergestellte Zwischenprodukt 2,2-Bis-(4-aminophenyl)-hexafluorpropan (4) kann nach einer neueren Literaturstelle (US-Patent 4 370 501 (1983)) auch aus 2,2-Bis-(4-hydroxyphenyl)-hexafluorpropan in einem dreistufigen Verfahren hergestellt werden, wobei man dieses zunächst mit einem 4-Chlorchinazolinderivat (10) der Formel

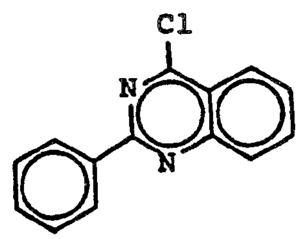

in Gegenwart von Kaliumhydroxid und Dimethylsulfoxid mit einem Kronenäther (18-Krone-6) als Katalysator unter Chlorwasserstoffabspaltung und Kaliumchloridbildung umsetzt. Der gebildete Diäther wird dann in inerter Atmosphäre bei etwa 320°C in einer zweiten Stufe zu dem entsprechenden Bis-chinazolinderivat umgelagert, das in einer dritten Stufe durch Behandlung mit wäßriger Kaliumhydroxidlösung und Äthylenglykol zum 2,2-Bis-(4-aminophenyl)-hexafluorpropan (4) gespalten wird.

Nach den Ausführungsbeispielen der US-PS 4 370 501 betragen die Ausbeuten in der 1. Stufe 65,5%, in der 2. Stufe 83,5% und in der 3. Stufe 30,2% d.Th..

Bezogen auf 2,2-Bis-(4-hydroxyphenyl)-hexafluorpropan liegt die Ausbeute bei diesem schon als preiswerten und mit "hohen Ausbeuten" beschriebenen Verfahren zur Herstellung von Anilinen and Dianilinen (s. US-PS 4 370 501, Spalte 4) lediglich bei 16,5%.

Dagegen wird in der Literatur (K.S.Y. Lau et al., Journal of Polymer Science, Polymer Chemistry Edition, 20, 2381-2393 (1982)) ein Verfahren beschrieben, das in sehr hohen Ausbeuten die Herstellung eines zu (4) isomeren Diamins, des 2,2-Bis-(3-aminophenyl)-hexafluorpropans (6), ermöglicht. Dazu wird ebenfalls vom 2,2-Bis-(4-hydroxyphenyl)-hexafluorpropan ausgegangen, dessen Hydroxylgruppen durch Umsetzung mit Tri-

fluormethansulfonsäureanhydrid in $F_3C-SO_3$-Gruppen übergeführt werden. Aus dieser Verbindung wird in einer zweiten Stufe durch katalytische Hydrierung mit einem Palladium-Kohle-Katalysator, der in Triäthylamin aufgeschlämmt ist, Trifluormethansulfonsäure abgespalten und so 2,2-Bisphenyl-hexafluorpropan erhalten. Diese Verbindung wird in einer dritten Stufe mit Salpetersäure in konzentrierter Schwefelsäure zum 2,2-Bis-(3-nitrophenyl)-hexafluorpropan nitriert, das dann in einer vierten Stufe an einem Palladium-Kohle-Katalysator zum 2,2-Bis-(3-aminophenyl)-hexafluorpropan (6) hydriert wird.

Nach den Ausführungsbeispielen betragen die Ausbeuten für die einzelnen Stufen in der 1. Stufe 96,5%, in der 2. Stufe 87,6%, in der 3. Stufe 90,0% und in der 4. Stufe 92,0% der Theorie.

Die Ausgangsverbindung für die beiden letztgenannten Verfahren – 2,2-Bis-(4-hydroxyphenyl)-hexafluorpropan (Bisphenol AF) – ist eine gängige Chemikalie und kann z.B. durch Umsetzung von Hexafluoraceton mit Phenol in flüssigem Fluorwasserstoff erhalten werden.

Ein Vergleich der beiden letztgenannten Verfahren macht deutlich, daß sich das m-Diamin (6) bei gleichem Ausgangsmaterial und trotz eines zusätzlichen Reaktionsschritts in wesentlich höherer Ausbeute – 70% gegenüber 16,5% – herstellen läßt als das p-Diamin (4).

In dem Bestreben, ein einfacheres und wirtschaftlicheres- auch in technischem Maßstabe mit Vorteil durchführbares- Verfahren zur Herstellung von 2,2-Bis-(3,4-diaminophenyl)hexafluorpropan (1) bereitzustellen, wurde nun gefunden, daß sich dieses auch ausgehend von 2,2-Bis-(3-aminophenyl)hexafluorpropan (6) herstellen läßt.

Erfindungsgegenstand ist daher ein Verfahren zur Herstellung von 2,2-Bis-(3,4-diaminophenyl)-hexafluorpropan (1), das dadurch gekennzeichnet ist, daß man

a) 2,2-Bis-(3-aminophenyl)-hexafluorpropan unter üblichen Acylierungsbedingungen, insbesondere durch Umsetzung mit Acetanhydrid, in eine acylierte Verbindung, deren Acylrest frei von aromatischen Resten ist, insbesondere 2,2-Bis-(3-acetamidophenyl)-hexafluorpropan (7), überführt,

b) diese unter üblichen Nitrierungsbedingungen, insbesondere mit Salpetersäure, zu 2,2-Bis-(3-acylamido-4-nitrophenyl)-hexafluorpropan nitriert, insbesondere zu 2,2-Bis-(3-acetamido-4-nitrophenyl)-hexafluorpropan (8),

c) das erhaltene Produkt unter für die Deacylierung üblichen Bedingungen, z.B. im alkalischen oder sauren Bereich, zu 2,2-Bis-(3-amino-4-nitrophenyl)-hexafluorpropan (9) deacyliert und

d) dieses unter für die Reduktion von Nitroverbindungen üblichen Bedingungen zu 2,2-Bis-(3,4-diaminophenyl)-hexafluorpropan (1) reduziert.

Diese Verbindung (1) wird isoliert oder – bevorzugt unter Ausschluß von Sauerstoff – in die Lösung eines ihrer Salze, vorzugsweise eines Hydrohalogenids, insbesondere des Hydrochlorids, übergeführt ; aus dieser werden Verunreinigungen durch ein Adsorptionsmittel, vorzugsweise Aktivkohle, entfernt und die Verbindung (1) unter Sauerstoffausschluß mit einer Base, wie Alkalihydroxyden, Erdalkalihydroxyden oder Alkalicarbonaten, vorzugsweise wäßriger Ammoniaklösung, ausgefällt. Auf diese Weise läßt sich die Verbindung (1) als hochreiner Feststoff erhalten.

Führt man die Stufe c) mit etwa 1-molarer methanolischwäßriger Natronlauge und die Stufe d) an einem Palladium-Kohle-Katalysator durch, so betragen die Ausbeuten

in der Stufe a) bis 99%,
in der Stufe b) bis 88%,
in der Stufe c) bis 87% und
in der Stufe d) bis 89% d.Th..

Bei der Ausbeuteangabe der Stufe d) ist die Reinigung des Tetramins (1) über das Hydrochlorid bereits berücksichtigt. Man erhält dabei einen weißen Feststoff, der laut Gaschromatographie eine Reinheit von mehr als 99,9% besitzt. Die hohe Reinheit des Feststoffes (1) ist insbesondere für seine Verwendung als Monomerkomponente zur Herstellung von Polybenzimidazolen wichtig.

Die Reinigung des Tetramins (1), das nach der Reduktion braungelb ist, über eines seiner Salze erfolgt zweckmäßig unter Schutzgas (z.B. Stickstoff), da (1) in gelöster Form sehr oxidationsempfindlich ist.

Das erfindungsgemäße Verfahren beruht zwar in seinen einzelnen Verfahrensstufen auf bekannten oder zu bekannten Verfahren analogen Reaktionen, führt aber in den Verfahrensstufen a), b) und c) zu neuen Zwischenprodukten, die in hohen Ausbeuten erhalten werden. Es stellt daher in seiner Gesamtheit ebenso wie die neuen Zwischenprodukte, die ebenfalls Gegenstand der Erfindung sind, und die Reinigung des 2,2-Bis-(3,4-diaminophenyl)-hexafluorpropans über eines seiner Salze einen erheblichen Fortschritt dar und eröffnet einen vorteilhaften Weg zur Herstellung dieser Verbindung.

Als Acylierungsmittel kommen im Prinzip alle üblichen Stoffe, z.B. Säurehalogenide oder Säureanhydride, in Frage, wobei im allgemeinen bei Temperaturen von 0-50°C, vorzugsweise bei 5-20°C gearbeitet wird. Bevorzugt ist in den Acylresten $R^1$ an der CO-Gruppe ein Kohlenwasserstoffrest mit 1 bis 6 Kohlenstoffatomen gebunden, also z.B. Alkyl, wie Methyl, Äthyl und die verschiedenen Propyl-, Butyl-, Pentyl- und Hexylreste, der –

gegebenenfalls durch Methyl substituierte – Cyclopentylrest und der Cyclohexylrest.

Für die Nitrierung der acylierten Verbindung können übliche Nitriersäuregemische, z.B. Salpetersäure/Schwefelsäure, Salpetersäure/Eisessig, Salpetersäure/Essigsäureanhydrid oder Salpetersäure-Wassergemische, benutzt werden. Insbesondere eignet sich eine 85 bis 95 gewichtsprozentige Salpetersäure zur Nitrierung, wobei die acylierte Verbindung zweckmäßig in die Salpetersäure eingetragen wird, vorzugsweise bei 0-40°C.

Die Entfernung der Acylgruppen kann nach den üblichen Deacylierungsmethoden durchgeführt werden. Im alkalischen Medium eignen sich besonders Lösungen in Wasser und/oder mit Wasser mischbaren Lösungsmitteln, insbesondere Alkoholen, die 5-50 Gew.-% einer Base, z.B. Alkalihydroxid oder Ammoniumbasen, enthalten. Die Deacylierung erfolgt im allgemeinen bei Temperaturen von 0-100°C. Als Alkohole kommen vor allem Methanol und Äthanol in Frage.

Die Reduktion der Nitrogruppen kann z.B. nach den üblichen katalytischen Methoden mit Hydrierungskatalysatoren eines Übergangsmetalls, insbesondere der VIII. Gruppe des Periodischen Systems nach Meyer-Mendelejew, oder nach stöchiometrischen Methoden (z.B. mit Zinn(II)chlorid/Eisessig) durchgeführt werden. Für die katalytischen Reduktionen können z.B. Platinmetalle, Kupfer, Eisen, Kobalt, Nickel, Metalloxide oder gemischte Metallkatalysatoren, vorzugsweise ohne oder mit Anwendung von Überdruck, in üblichen Verdünnungsmitteln wie Alkoholen, aromatischen Kohlenwasserstoffen (z.B. Toluol), Estern oder ähnlichen organischen Lösungsmitteln bei Temperaturen von z.B. 10-80°C eingesetzt werden.

Zwecks Reinigung kann die Verbindung (1) bei z.B. 10-100°C in Wasser in eines ihrer wasserlöslichen Salze (z.B. Halogenid, Hydrogensulfat) übergeführt werden, das gegenüber Aminogruppen unter den angewandten Bedingungen inert ist.

Die bevorzugten neuen Zwischenprodukte haben die Formel II (siehe Patentanspruch 1), worin $R^1$ die oben angegebene Bedeutung hat und $R^2$ Wasserstoff oder $NO_2$ darstellt oder worin $R^1$ Wasserstoff und $R^2$ $NO_2$ ist. Von den Verbindungen, in denen $R^1$ Acyl darstellt, sind die besonders bevorzugt, in denen $R^1$ Acetyl ist. Mit anderen Worten sind neben dem 2,2-Bis-(3-amino-4-nitrophenyl)-hexafluorpropan (9) das 2,2-Bis-(3-acetamidophenyl)-hexafluorpropan (7) und das 2,2-Bis-(3-acetamido-4-nitrophenyl)-hexafluorpropan (8) bevorzugt.

Beispiele

**1) 2,2-Bis-(3-acetamidophenyl)-hexafluorpropan (7)**

66,8 g (0,2 mol) 2,2-Bis-(3-aminophenyl)-hexafluorpropan werden in 250 ml Eisessig gelöst und mit 130 ml Eiswasser versetzt. Bei 5°C werden 48,1 g (1,3 mol) Acetanhydrid zugegeben und es wird 30 Minuten gerührt. Der ausgefallene Niederschlag wird abgesaugt, intensiv mit 500 ml Wasser gewaschen und bei 80°C getrocknet. Ausbeute 83 g (0,198 mol) (7) ; weißer Feststoff ; Fp. 309-311°C.
Analysenwerte in % :
$C_{19}H_{16}F_6N_2O_2$ ber. : C 54,55, H 3,86, N 6,70, F 27,25, O 8,90
(418,34) gef. : C 54,1, H 3,8, N 6,5, F 26,7, O 9,2
$^1$H-NMR ($d^6$-DMSO) δ(ppm) : 10,1 s 2 NH, 7,9-6,95 m 8 H, 2,05 s 2 $CH_3$
$^{19}$F-NMR ($d^6$-DMSO) δ(ppm) : –62,4 s $CF_3$
IR (KBr) γ : 3300 $cm^{-1}$ NH, 1670 $cm^{-1}$ C=0 1260-1140 $cm^{-1}$ $CF_3$

**2) 2,2-Bis-(3-acetamido-4-nitrophenyl)-hexafluorpropan (8)**

83,7 g (0,2 mol) (7) werden bei 20-30°C portionsweise in 400 ml 95 gewichtsprozentige Salpetersäure eingetragen. Die Reaktionsmischung wird 30 Minuten nachgerührt und anschließend auf 1000 g einer Mischung aus Wasser und Eis gegossen. Der Niederschlag wird abgefiltert und mit Wasser gewaschen, bis ein pH-Wert von 3 erreicht ist. Das getrocknete Rohprodukt wird in 250 ml Äthanol suspendiert und 5 Minuten unter Rückfluß gekocht. Nach dem Erkalten auf 25°C wird der Feststoff abfiltriert und bei 100°C getrocknet. Ausbeute 89 g (0,18 mol) ; gelber Feststoff ; Fp. 187-189°C. Analysenwerte in % :
$C_{19}H_{14}F_6N_4O_6$ ber. : C 44,89, H 2,87, F 22,43, N 11,02, O 18,89
((508,33) gef. : C 45,2, H 2,7, F 22,0, N 11,3, O 18,9
$^1$H-NMR ($CDCl_3$) δ(ppm) : 10,3 s 2 NH, 9,0 s 2 H, 8,25 d 2 H, 7,25 d 2 H, 2,3 s 2 $CH_3$
$^{19}$F NMR ($CDCl_3$) δ(ppm) : –63,7 s $CF_3$

**3) 2,2-Bis-(3-amino-4-nitrophenyl)-hexafluorpropan (9)**

160 g (0,31 mol) (8) werden in einer Lösung aus 65 ml Wasser, 570 ml Methanol und 25 g Natriumhydroxid

15 Minuten lang unter Rückfluß erhitzt. Die heiße Lösung wird mit 1300 ml heißem Wasser verdünnt und anschließend auf 0-5°C abgekühlt. Der Feststoff wird abgesaugt, mit Wasser gewaschen, bei 80°C getrocknet und aus Toluol umkristallisiert. Ausbeute 115 g (0,27 mol) ; gelber Feststoff ; Fp. 257-259°C. Analysenwerte in % :

$C_{15}H_{10}F_6N_4O_4$ ber. : C 42,46, H 2,38, F 26,87, N 13,21, O 15,09
(424,26) gef. : C 42,7, H 2,3, F 27,2, N 13,6, O 15,5
$^1$H-NMR ($d^6$-DMSO) δ(ppm) : 8,2 d 2 H, 7,8 s 2 $NH_2$, 7,4 s 2 H, 6,7 d 2 H
$^{19}$F-NMR ($d^6$-DMSO) δ(ppm) : −62,1 s $CF_3$

**4) 2,2-Bis-(3,4-diaminophenyl)-hexafluorpropan (1)**

96,7 g (0,23 mol) (9) werden in 1000 ml Essigsäureäthylester gelöst und in einem Autoklaven nach der Zugabe von 3 g eines Palladium-Kohle-Katalysators (5% Pd) bei 25°C mit Wasserstoff (100 bar) reduziert. Nach dem Abfiltrieren des Katalysators wird der Essigester am Rotationsverdampfer abgetrennt. Der Rückstand wird mit 1 l Wasser aufgenommen und mit halbkonzentrierter Salzsäure auf einen pH-Wert von 1 eingestellt. Anschließend wird auf 70-80°C erhitzt, mit 20 g Aktivkohle versetzt, 15 Minuten bei 70-80°C gerührt und die Aktivkohle abfiltriert. Das farblose Filtrat wird mit Stickstoff gesättigt und bei 10-20°C unter Schutzgas mit halbkonzentrierter Ammoniaklösung auf einen pH-Wert von 7 eingestellt. Der Niederschlag wird abgetrennt, kräftig mit Wasser gewaschen und unter vermindertem Druck bis zur Gewichtskonstanz getrocknet. Ausbeute 74 g (0,20 mol) ; gaschromatographische Reinheit über 99,9%, weißer Feststoff ; Fp. 218-220°C. Analysenwerte in % :

$C_{15}H_{14}F_6N_4$ : ber. : C 49,45, H 3,87, N 15,38, F 31,29
(364,29) gef. : C 49,4, H 3,7, N 15,8, F 30,9
$^1$H-NMR ($d^6$-DMSO) δ(ppm) : 6,4-6,5 m 6 H, 4,6 s 4 $NH_2$
$^{19}$F-NMR ($d^6$-DMSO) δ(ppm) : −62,44 s $CF_3$
IR (KBr) γ : 3430 und 3350 $cm^{-1}$ ($-NH_2$), 1260-1130 $cm^{-1}$ ($-CF_3$)

## Ansprüche

1. Verbindungen der Formel

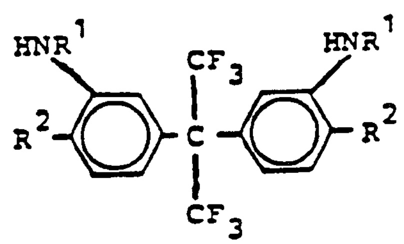

worin $R^1$ Wasserstoff oder einen Acylrest darstellt, in dem an der CO-Gruppe ein nicht-aromatischer Kohlenwasserstoffrest mit 1 bis 6 C-Atomen gebunden ist und $R^2$ dann, wenn $R^1$ einen Acylrest darstellt, H oder $NO_2$ ist und, wenn $R^1$ Wasserstoff ist, $NO_2$ ist.

2. Verbindung nach Anspruch 1, dadurch gekennzeichnet, daß $R^1$ der Acetylrest und $R^2$ H oder $NO_2$ ist.

3. Verbindung nach Anspruch 1, dadurch gekennzeichnet, daß $R^1$ H und $R^2$ $NO_2$ ist.

4. Verfahren zur Herstellung von 2,2-Bis-(3,4-diaminophenyl)-hexafluorpropan der Formel

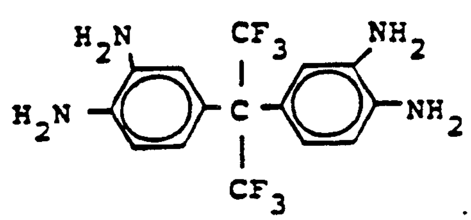

dadurch gekennzeichnet, daß man

a) 2,2-Bis-(3-aminophenyl)-hexafluorpropan unter üblichen Acylierungsbedingungen zu einem 2,2-Bis-(3-acylamidophenyl)-hexafluorpropan acyliert, deren Acylrest frei von aromatischen Resten ist,

b) das so erhaltene 2,2-Bis-(3-acylamidophenyl)-hexafluorpropan unter üblichen Nitrierungsbedingungen zu 2,2-Bis-(3-acylamido-4-nitrophenyl)-hexafluorpropan nitriert,

c) das so erhaltene 2,2-Bis-(3-acylamido-4-nitrophenyl)-hexafluorpropan unter für die Deacylierung üblichen Bedingungen zu 2,2-Bis-(3-amino-4-nitrophenyl)-hexafluorpropan deacyliert,

d) dieses unter für die Reduktion von Nitroverbindungen üblichen Bedingungen zu 2,2-Bis-(3,4-diaminophenyl)-hexafluorpropan reduziert und diese Verbindung isoliert oder einer Reinigung in der Weise unterwirft, daß man sie in die Lösung eines ihrer Salze überführt, Verunreinigungen an einem Adsorptionsmittel adsorbiert und das 2,2-Bis-(3,4-diaminophenyl)-hexafluorpropan mit einer Base ausfällt.

5. Verfahren nach Anspruch 4, dadurch gekennzeichnet, daß die Acylierung mit einem Säurehalogenid oder Säureanhydrid durchgeführt wird, in dem an die CO-Gruppe ein Kohlenwasserstoffrest mit 1 bis 6 C-Atomen gebunden ist, wobei die Acylierung bevorzugt eine Acetylierung ist und vorzugsweise mit Acetanhydrid durchgeführt wird.

6. Verfahren nach Anspruch 4 oder 5, dadurch gekennzeichnet, daß die Reduktion in der Stufe d) katalytisch mit Wasserstoff an einem Übergangsmetall, insbesondere der VIII. Gruppe des Periodischen Systems, oder nach einer stöchiometrischen Methode durchgeführt wird.

7. Verfahren nach einem oder mehreren der Ansprüche 4 bis 6, gekennzeichnet durch wenigstens eines der Merkmale, daß

a) die Acylierung bei 0-50°C, vorzugsweise bei 5 bis 20°C durchgeführt wird,

$b_1$) die Nitrierung mit einer 85 bis 95 %igen Salpetersäure durchgeführt wird,

$b_2$) bei der Nitrierung die acylierte Verbindung in das Salpetersäure-Wasser-Gemisch, vorzugsweise bei 0 bis 40°C eingetragen wird,

$c_1$) die Deacylierung in einer Lösung in Wasser und/oder mit Wasser mischbaren Lösungsmitteln, insbesondere Alkoholen, durchgeführt wird, die 5 bis 50 Gew.-% einer Base enthalten,

$c_2$) die Deacylierung bei Temperaturen von 0 bis 100°C erfolgt,

$d_1$) das in der Stufe d) erhaltene 2,2-Bis-(3,4-diaminophenyl)-hexafluorpropan unter Ausschluß von Sauerstoff in die Lösung eines seiner Salze übergeführt wird,

$d_2$) die Überführung in ein Hydrohalogenid, insbesondere das Hydrochlorid, erfolgt,

$d_3$) das Adsorptionsmittel Aktivkohle ist und

$d_4$) das 2,2-Bis-(3,4-diaminophenyl)-hexafluorpropan mit wäßriger Ammoniaklösung ausgefällt wird.

8. Verfahren nach einem oder mehreren der Ansprüche 4 bis 7, dadurch gekennzeichnet, daß die Ausfällung des 2,2-Bis(3,4-diaminophenyl)-hexafluorpropans mit der Base unter Sauerstoffausschluß ausgeführt werden.

## Claims

1. A compound of the formula

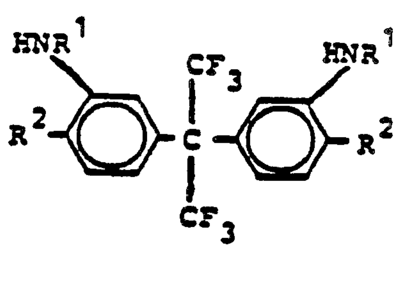

in which $R^1$ is hydrogen or an acyl radical in which a non-aromatic hydrocarbon radical having 1 to 6 carbon atoms is bonded to the CO group and $R^2$ is H or $NO_2$ when $R^1$ is an acyl radical, and is $NO_2$ when $R^1$ is hydrogen.

2. The compound as claimed in claim 1, wherein $R^1$ is the acetyl radical and $R^2$ is H or $NO_2$.

3. The compound as claimed in claim 1, wherein $R^1$ is H and $R^2$ is $NO_2$.

4. A process for the preparation of 2,2-bis-(3,4-diaminophenyl)hexafluoropropane of the formula

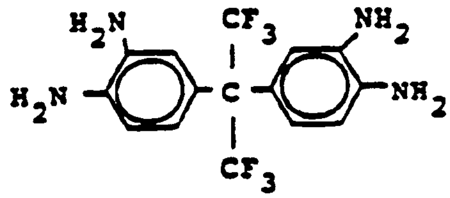

which comprises

a) acylating 2,2-bis-(3-aminophenyl)hexafluoropropane to give a 2,2-bis-(3-acylamidophenyl)hexafluoropropane, the acyl radical of which is free of aromatic radicals, under customary acylation conditions,

b) nitrating the 2,2-bis-(3-acylamidophenyl)hexafluoropropane thus obtained under customary nitration conditions to give 2,2-bis-(3-acylamido-4-nitrophenyl)hexafluoropropane,

c) deacylating the 2,2-bis-(3-acylamido-4-nitrophenyl)-hexafluoropropane thus obtained under the customary conditions for deacylation to give 2,2-bis-(3-amino-4-nitrophenyl)hexafluoropropane,

d) reducing this under customary conditions for the reduction of nitro compounds to give 2,2-bis-(3,4-diaminophenyl)hexafluoropropane and isolating this compound or subjecting it to purification by converting it into one of its salts in the solution, adsorbing the impurities on an adsorbent and precipitating the 2,2-bis-(3,4-diaminophenyl)hexafluoropropane using a base.

5. The process as claimed in claim 4, wherein the acylation is carried out using an acid halide or acid anhydride in which a hydrocarbon radical having 1 to 6 carbon atoms is bonded to the CO group, the acylation preferably being an acetylation and preferably being carried out using acetic anhydride.

6. The process as claimed in claim 4 or 5, wherein the reduction in step d) is carried out catalytically using hydrogen on a transition metal, in particular of group VIII of the periodic table, or by a stoichiometric method.

7. The process as claimed in one or more of claims 4 to 6, comprising at least one of the features

a) the acylation is carried out at 0-50°C, preferably at 5 to 20°C,

$b_1$) the nitration is carried out using an 85 to 95% strength nitric acid,

$b_2$) the acylated compound is added to the nitric acid-water mixture, preferably at 0 to 40°C, during the nitration,

$c_1$) the deacylation is carried out in a solution in water and/or water-miscible solvents, in particular alcohols, which contain 5 to 50% by weight of a base,

$c_2$) the deacylation is carried out at temperatures from 0 to 100°C,

$d_1$) the 2,2-bis-(3,4-diaminophenyl)hexafluoropropane obtained in step d) is converted into the solution of one of its salts with exclusion of oxygen,

$d_2$) conversion into a hydrohalide, in particular the hydrochloride, is carried out,

$d_3$) the adsorbing agent is active carbon and

$d_4$) the 2,2-bis-(3,4-diaminophenyl)hexafluoropropane is precipitated using aqueous ammonia solution.

8. The process as claimed in one or more of claims 4 to 7, wherein the precipitation of the 2,2-bis-(3,4-diaminophenyl)hexafluoropropane using the base is carried out with exclusion of oxygen.

## Revendications

1. Composés répondant à la formule

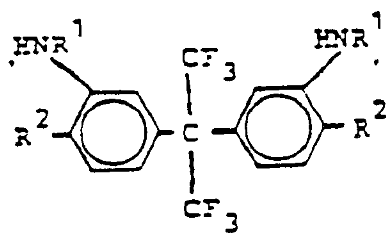

dans laquelle $R^1$ représente l'hydrogène ou un radical acyle dont le radical CO est lié à un radical hydrocarboné non aromatique contenant de 1 à 6 atomes de carbone, et $R^2$ représente H ou $NO_2$ lorsque $R^1$ désigne un radical acyle, et un radical $NO_2$ lorsque $R^1$ représente l'hydrogène.

2. Composé selon la revendication 1 caractérisé en ce que $R^1$ représente un radical acétyle et $R^2$ repré-

sente H ou $NO_2$.

3. Composé selon la revendication 1 caractérisé en ce que $R^1$ représente H et $R^2$ représente $NO_2$.

4. Procédé pour préparer le bis-(diamino-3,4 phényl)-2,2 hexafluoropropane, composé qui répond à la formule suivante :

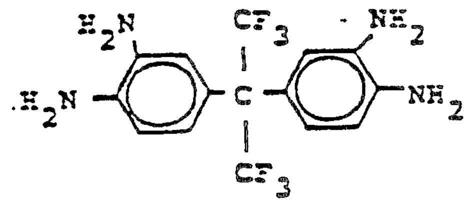

procédé caractérisé en ce que

a) on acyle le bis-(amino-3 phényl)-2,2 hexafluoropropane dans des conditions d'acylation usuelles pour obtenir un bis-(acylamino-3 phényl)-2,2 hexafluoropropane dont le radical acyle est dépourvu de radical aromatique,

b) on nitre le bis-(acylamino-3 phényl)-2,2 hexafluoropropane ainsi obtenu, dans des conditions de nitration usuelles, pour obtenir le bis-(acylamino-3- nitro-4 phényl)-2,2 hexafluoropropane,

c) on désacyle le bis-(acylamino-3- nitro-4 phényl)-2,2 hexafluoropropane ainsi obtenu, dans des conditions de désacylation usuelles, pour obtenir le bis-(amino-3 nitro-4 phényl)-2,2 hexafluoropropane,

d) on réduit celui-ci, dans des conditions habituellement appliquées pour la réduction de composés nitrés, pour obtenir le bis-(diamino-3,4 phényl)-2,2 hexafluoropropane, et on isole ce composé, ou on le soumet à une purification consistant à le transformer en une solution d'un de ses sels, à adsorber les impuretés sur un agent d'adsorption et à faire précipiter le bis-(diamino-3,4 phényl)-2,2 hexafluoropropane au moyen d'une base.

5. Procédé selon la revendication 4 caractérisé en ce qu'on effectue l'acylation au moyen d'un halogénure d'acide ou d'un anhydride d'acide dans lequel le radical CO est lié à un radical hydrocarboné contenant de 1 à 6 atomes de carbone, l'acylation étant de préférence une acétylation et étant de préférence effectuée au moyen de l'anhydride acétique.

6. Procédé selon l'une des revendications 4 et 5, caractérisé en ce que la réduction de l'étape d) est effectuée catalytiquement au moyen d'hydrogène en présence d'un métal de transition, plus particulièrement d'un métal du huitième groupe de la classification périodique, ou par une méthode stoechiométrique.

7. Procédé selon l'une quelconque des revendications 4 à 6, caractérisé par au moins une des particularités suivantes :

a) on effectue l'acylation à 0-50°C, de préférence à 520°C,

$b_1$) on effectue la nitration avec un acide nitrique d'une concentration comprise entre 85 et 95%,

$b_2$) au cours de la nitration on introduit le composé acylé dans le mélange d'acide nitrique et d'eau, de préférence à une température de 0 à 40°C,

$c_1$) on effectue la désacylation sur une solution dans de l'eau et/ou dans des solvants miscibles à l'eau, plus particulièrement dans des alcools, qui contiennent de 5 à 50% en poids d'une base,

$C_2$) on effectue la désacylation à des températures de 0 à 100°C,

$d_1$) le bis-(diamino-3,4 phényl)-2,2 hexafluoropropane obtenu à l'étape d) est transformé, à l'abri de l'oxygène, en une solution d'un de ses sels,

$d_2$) la transformation est une transformation en un halogénhydrate, plus particulièrement en le chlorhydrate,

$d_3$) l'agent d'adsorption est du charbon actif, et

$d_4$) on fait précipiter le bis-(diamino-3,4 phényl)-2,2 hexafluoropropane avec une solution ammoniacale aqueuse.

8. Procédé selon l'une quelconque des revendications 4 à 7, caractérisé en ce que la précipitation du bis-(diamino-3,4 phényl)-2,2 hexafluoropropane par la base est effectuée à l'abri de l'oxygène.